# EUROPEAN PATENT SPECIFICATION

(11) **EP 4 337 042 B1**
(45) Date of publication and mention of the grant of the patent: **29.01.2025**
(21) Application number: 22728452.8
(22) Date of filing: 09.05.2022
(51) Int. Cl.: A24F 40/40, A24F 40/20, A24F 40/46, A24D 1/20, A61M 11/04, A61M 15/00, A61M 15/06

(54) **AEROSOL GENERATING DEVICE COMPRISING A CUP-SHAPED HEATING CHAMBER DEFINING AN OPEN END AND A SEALED END**
AEROSOLERZEUGUNGSVORRICHTUNG MIT EINER BECHERFÖRMIGEN HEIZKAMMER MIT EINEM OFFENEN UND EINEM GESCHLOSSENEN ENDE
DISPOSITIF DE GÉNÉRATION D'AÉROSOL COMPRENANT UNE CHAMBRE DE CHAUFFAGE EN FORME DE COUPELLE AYANT UNE EXTRÉMITÉ OUVERTE ET UNE EXTRÉMITÉ FERMÉ

(30) Priority: 10.05.2021 EP 21173025
(43) Date of publication of application: 20.03.2024
(73) Proprietor: JT International S.A., 1202 Genève (CH)
(72) Inventor: AKIYAMA, Takeshi, TOKYO, 130--8603 (JP)
(74) Representative: Lavoix
(86) International application number: PCT/EP2022/062498
(87) International publication number: WO 2022/238337

(56) References cited:
- EP-A1- 3 711 592
- EP-A2- 3 462 939
- EP-B1- 3 462 939
- WO-A1-2020/074604
- WO-A1-2020/239599
- US-B2- 9 380 813

## Description

### FIELD OF THE INVENTION

The present invention concerns an aerosol generating device comprising a cup-shaped heating chamber defining an open end and a sealed end.

The aerosol generating device according to the invention is configured to operate with an aerosol generating substrate which presents for example a solid substrate able to form aerosol when being heated. Thus, such type of aerosol generating devices, also known as heat-not-burn devices, is adapted to heat, rather than burn, the substrate by conduction, convection and/or radiation, to generate aerosol for inhalation.

### BACKGROUND OF THE INVENTION

The popularity and use of reduced-risk or modified-risk devices (also known as vaporisers) has grown rapidly in the past few years as an aid to assist habitual smokers wishing to quit smoking traditional tobacco products such as cigarettes, cigars, cigarillos, and rolling tobacco. Various devices and systems are available that heat or warm vaporizable substances as opposed to burning tobacco in conventional tobacco products.

A commonly available reduced-risk or modified-risk device is the heated substrate aerosol generation device or heat-not-burn device. Devices of this type generate aerosol or vapour by heating an aerosol substrate that typically comprises moist leaf tobacco or other suitable vaporizable material to a temperature typically in the range 150°C to 350°C. Heating an aerosol substrate, but not combusting or burning it, releases aerosol that comprises the components sought by the user but not the toxic and carcinogenic byproducts of combustion and burning. Furthermore, the aerosol produced by heating the tobacco or other vaporizable material does not typically comprise the burnt or bitter taste resulting from combustion and burning that can be unpleasant for the user and so the substrate does not therefore require the sugars and other additives that are typically added to such materials to make the smoke and/or vapour more palatable for the user.

In order to be able to form aerosol while heating an aerosol forming substrate, the aerosol forming devices define generally an airflow path extending between a flow inlet and a flow outlet. The flow outlet may be arranged on a mouthpiece of the device which is intended to be in contact with the user's lips and/or mouth to inhale the aerosol.

WO 2020/074604 discloses an aerosol forming device.

During operation of an aerosol forming device, its surface temperature can achieve an important value and may even burn the user holding the device. Furthermore, in some cases, the user can erroneously blow into the flow outlet instead of inhaling the vapour. In this case, the flow direction can be reversed and the flow will exit by the flow inlet and may thus burn the user. Finally, in some cases, fresh air entering through the flow inlet may be disrupted. This can deteriorate the performances of aerosol generation.

### SUMMARY OF THE INVENTION

One of the aims of the invention is to provide an aerosol generating device minimizing the risk of user injury and presenting increased performances of aerosol generation.

For this purpose, the invention, as defined in claim 1, relates to an aerosol generating device configured to operate with an aerosol generating substrate and comprising:
- a device body extending along a device axis and defining at least one side wall extending along the device axis;
- a cup-shaped heating chamber defining an open end and a sealed end opposite to the open end, the sealed end being air impermeable, the heating chamber being configured to receive a heater part of the aerosol generating substrate through the open end;
- an airflow path extending between an flow inlet and an flow outlet through the open end of the heating chamber, the flow inlet being arranged on the side wall of the device body.

By disposing the flow inlet on a side wall of the device, it is possible to prevent hot vapour from burning the user when blowing by mistake. Additionally, it makes it possible to arrange the airflow path at least partially along the side wall of the device. This decreases the surface temperature of this wall. Thus, the user can hold the device without risk of burning. Finally, the arrangement of the flow inlet prevents the risk of masking the inlet for example by the user's hand or a finger. Thus, fresh air can easily enter to the airflow path which enhances the substrate heating effect as convection heating due to getting energy.

According to some embodiments, the flow inlet is formed in a transition zone between the mouthpiece and the housing.

According to some embodiments, the flow inlet is formed in a transition zone between the mouthpiece and the housing.

Thanks to these features, the flow inlet can be advantageously arranged in a zone of the side wall of the device which has a very little risk to be masked for example with the user's hand or finger while holding the device. Additionally, according to this arrangement, a reversed flow due for example to user blowing is guided in the direction opposite to the user.

According to some embodiments, the mouthpiece and the housing form a single piece, the transition zone being formed by a smooth surface extending from the mouthpiece to the housing.

Thanks to these features, an aerosol generating substrate can be loaded directly from an opening formed in the mouthpiece.

According to some embodiments, the housing defines an insertion opening extending perpendicularly to the device axis and configured to receive the mouthpiece.

Thanks to these features, the mouthpiece can be removable and eventually replaceable. Additionally, due to such an arrangement, an aerosol generating substrate can be loaded into the heating chamber when the mouthpiece is removed from the device. Then, the substrate can be fixed inside the heating chamber by fixing the mouthpiece on the housing.

According to some embodiments, the mouthpiece defines a recess portion forming the flow inlet when the mouthpiece is inserted into the insertion opening of the housing.

According to some embodiments, the through-hole of the mouthpiece opens to the recess portion.

Thanks to these features, the flow inlet can be easily formed by the space formed between the mouthpiece and the device body.

According to some embodiments, wherein the device body defines an insertion opening on one of its ends suitable to receive a part of the aerosol generating substrate;
when received in the insertion opening, the aerosol generating substrate forming a protruding part from the device body, said protruding part forming a mouthpiece.

Thanks to these features, a part of the aerosol generating substrate can be used as a mouthpiece.

According to some embodiments, the flow inlet is arranged to face ventilation holes of the aerosol generating substrate.

Thanks to these features, in the case where the aerosol generating substrate is provided with ventilation holes, these ventilation holes can face the flow inlet and thus be exposed to fresh air.

According to some embodiments, the airflow path comprises an upstream portion extending from the flow inlet until an abutting end of the aerosol generating substrate, and a downstream portion extending from said abutting end until the flow outlet through the aerosol generating substrate, when the aerosol generating substrate is inserted into the heating chamber;
preferably, the upstream portion of the airflow path extends through the open end of the heating chamber.

Thanks to these features, the airflow path can enter and exit by the same open end of the heating chamber. The heating chamber presents thus an air impermeable cup having in particular only one open end.

According to some embodiments, at least a part of the upstream portion of the airflow path extends through the heating chamber outside the aerosol generating substrate.

Thanks to these features, said part of the upstream portion can conduct fresh air from outside of the device and thus, decreases the surface temperature of the side wall.

According to some embodiments, the heating chamber extends along the device axis and defines a rectangular cross-sectional shape having a pair of chamber lateral walls facing each other and a pair of chamber contact walls facing each other and intended to be in contact with substrate contact walls of the aerosol generating substrate.

Thanks to these features, the aerosol generating substrate can be homogenously heated inside the heating chamber.

According to some embodiments, said part of the upstream portion of the airflow path extends between at least one chamber lateral wall and the aerosol generating substrate.

Thanks to these features, fresh air can be conducted inside the heating chamber until the abutting end of the aerosol generating substrate without passing through the substrate and then, from the abutting end inside the substrate. Thus, in the inlet portion of the aerosol generating substrate, the air is already pre-heated and the substrate can homogenously be impregnated with the hot air.

According to some embodiments, the sealed end of the heating chamber forms a transition portion between the upstream portion and the downstream portion of the airflow path.

According to some embodiments, the sealed end defines stopping means preventing contact of the abutting end of the aerosol generating substrate with a distal wall of the heating chamber sealing it at the sealed end;
preferably, the stopping means being formed by a rounded shape of the distal wall or a rib extending from the distal wall.

According to some embodiments, the sealed end defines stopping means adapted to form a space between the sealed end and a face of the aerosol generating substrate facing the sealed end when the aerosol generating substrate is inserted into the heating chamber.

According to some embodiments, the airflow path comprises a transition portion extending inside the space between the sealed end and the face of the aerosol generating substrate facing the sealed end when the aerosol generating substrate is inserted into the heating chamber. The transition portion may fluidically connect the upstream portion to the downstream portion of the airflow path. For example, the transition portion forms a turn of a flow direction between the upstream portion and the downstream portion having an angle substantially equal to 180 degrees.

According to some embodiments, the transition portion of the airflow path forms substantially a U shape in a cross section comprising the device axis.

Thanks to one or several of these features, the direction of the flow can be reversed inside the heating chamber at the sealed end. Thus, the flow can be forced to flow a "U" turn and enter inside the aerosol generating substrate.

According to some embodiments, the only air permeable opening of the heating chamber is formed at the open end.

The invention furthermore relates to an assembly comprising an aerosol generating device as described above, and an aerosol generating substrate.

According to some embodiments of the assembly, the aerosol generating device is configured to operate with the aerosol generating substrate of the assembly.

According to some embodiments of the assembly, the aerosol generating substrate is inserted into the heating chamber of the aerosol generating device.

### BRIEF DESCRIPTION OF THE DRAWINGS

The invention and its advantages will be better understood upon reading the following description, which is given solely by way of non-limiting example and which is made with reference to the appended drawings, in which:
- Figure 1 is a perspective view of an aerosol generating device according to a first embodiment;
- Figure 2 is a perspective view of an aerosol generating substrate usable with the aerosol generating device of Figure 1;
- Figure 3 is a perspective view of a mouthpiece of the aerosol generating device of Figure 1;
- Figure 4 is a perspective view of the mouthpiece of Figure 3 mounted on the housing of the aerosol generating device of Figure 1;
- Figure 5 is a perspective view of a heating chamber of the aerosol generating device of Figure 1, the heating chamber receiving the aerosol generating substrate of Figure 2;
- Figure 6 is a view similar to the view of Figure 5 where the heating chamber and the aerosol generating substrate are shown without the housing;
- Figure 7 is a cross-sectional view of Figure 6 according to plane VII;
- Figure 8 is a cross-sectional view of Figure 6 according to plane VIII;
- Figure 9 is a view similar to the view of Figure 8 showing another possible example of the heating chamber;
- Figure 10 is a perspective view of an aerosol generating device according to a second embodiment;
- Figure 11 is a perspective view of an aerosol generating device according to a third embodiment;
- Figure 12 is a cross-sectional view similar to the view of Figure 7 showing a preferred example of a heater part of the aerosol generating substrate.

### DETAILED DESCRIPTION OF THE INVENTION

Before describing the invention, it is to be understood that it is not limited to the details of construction set forth in the following description.

As used herein, the term "aerosol **generating** device" or "device" may include a vaping device to deliver an aerosol to a user, including an aerosol for vaping, by means of a heater element explained in further detail below. The device may be portable. "Portable" may refer to the device being for use when held by a user. The device may be adapted to generate a variable amount of aerosol, e.g. by activating the heater element for a variable amount of time (as opposed to a metered dose of aerosol), which can be controlled by a trigger. The trigger may be user activated, such as a vaping button and/or inhalation sensor. The inhalation sensor may be sensitive to the strength of inhalation as well as the duration of inhalation to enable a variable amount of vapour to be provided (so as to mimic the effect of smoking a conventional combustible smoking article such as a cigarette, cigar or pipe, etc.). The device may include a temperature regulation control to drive the temperature of the heater and/or the heated aerosol generating substance (aerosol pre-cursor) to a specified target temperature and thereafter to maintain the temperature at the target temperature that enables efficient generation of aerosol.

As used herein, the term "**aerosol**" may include a suspension of vaporizable material as one or more of: solid particles; liquid droplets; gas. Said suspension may be in a gas including air. Aerosol herein may generally refer to/include a vapour. Aerosol may include one or more components of the vaporizable material.

As used herein, the term "**vaporizable material**" or "**precursor**" may refer to a smokable material which may for example comprise nicotine or tobacco and an aerosol former. Tobacco may take the form of various materials such as shredded tobacco, granulated tobacco, tobacco leaf and/or reconstituted tobacco. Suitable aerosol formers include: a polyol such as sorbitol, glycerol, and glycols like propylene glycol or triethylene glycol; a non-polyol such as monohydric alcohols, acids such as lactic acid, glycerol derivatives, esters such as triacetin, triethylene glycol diacetate, triethyl citrate, glycerin or vegetable glycerin. In some embodiments, the aerosol generating agent may be glycerol, propylene glycol, or a mixture of glycerol and propylene glycol. The substrate may also comprise at least one of a gelling agent, a binding agent, a stabilizing agent, and a humectant.

### FIRST EMBODIMENT

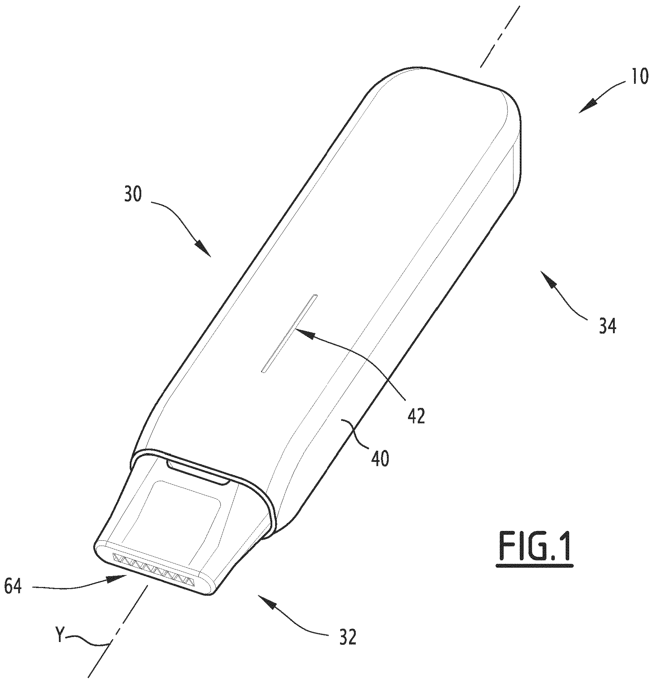

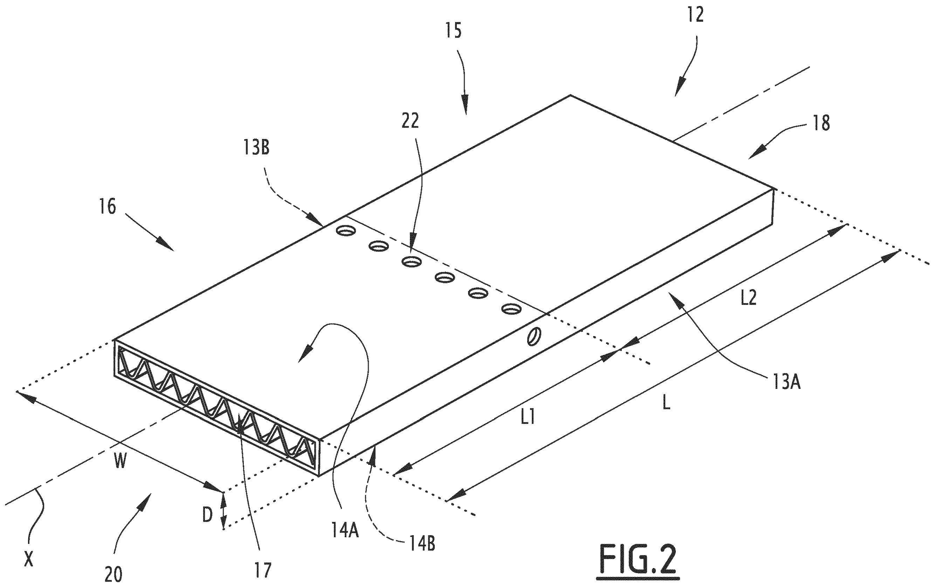

Figure 1 shows an aerosol generating device 10 according to the first embodiment. The aerosol generating device 10 is intended to operate with an aerosol generating substrate 12 shown in more detail on Figure 2.

An assembly comprises the aerosol generating device 10 and the aerosol generating substrate 12. The aerosol generating substrate 12 is in particular inserted into the aerosol generating device 10. For example, the assembly comprises the aerosol generating substrate 12 being inserted into a heating chamber 45 of the aerosol generating device 10. Embodiments of the heating chamber 45 are described below.

In reference to Figure 2, the aerosol generating substrate 12 is for example a flat-shaped cuboid extending along a substrate axis X and having external dimensions LxWxD. In a typical example, the length L of the substrate according to the substrate axis X equals substantially to 33 mm while its width W and depth D are substantially equal respectively to 12 mm and 1,2 mm. According to different examples, the values L, W and D can be selected within a range of +/- 40%, for example. The depth D of the substrate 12 is formed by a pair of parallel walls 13A, 13B, called hereinafter substrate lateral walls 13A, 13B, and the width W of the substrate is formed by a pair of parallel walls 14A, 14B, called hereinafter substrate contact walls 14A, 14B. According to other embodiments, the aerosol generating substrate 12 can have any other suitable shape and/or external dimensions. For example, the aerosol generating substrate 12 may form a circular tube shape.

The aerosol generating substrate 12 comprises a heater part 15 and a mouthpiece part 16 arranged along the substrate axis X. In some embodiments, the aerosol generating substrate 12 may comprise only the heater part 15. The heater part 15 may for example be slightly longer than the mouthpiece part 16. For example, the length L2 of the heater part 15 according to the substrate axis X may be substantially equal to 18 mm and the length L1 of the mouthpiece part 16 according to the substrate axis X may be substantially equal to 15 mm. The heater part 15 defines an abutting end 18 of the substrate 12 and the mouthpiece part 16 defines a mouth end 20 of the substrate 12. The heater part 15 and the mouthpiece part 16 may be fixed one to the other by a unique wrapper extending around the substrate axis X. In other embodiments, the parts 15, 16 may be wrapped by different wrappers and fixed one to the other by any other suitable mean. The or each wrapper may, for example, comprise paper and/or non-woven fabric and/or aluminium. The or each wrapper may be porous or air impermeable. The or each wrapper forms a plurality of airflow channels extending inside the substrate 12 between the abutting end 18 and the mouth end 20.

The heater part 14 is intended to be heated by a heater (using a heating chamber in the present example) and comprises vaporizable material as defined above. According to the first and the second embodiments, the mouthpiece part 16 is intended to be received inside a mouthpiece as it will be explained in further detail below. According to other embodiments, the mouthpiece part 16 forms itself a mouthpiece intended to be in contact with the user's mouth and/or lips. The mouthpiece part 16 comprises a core 17 acting for example like a filter. The core 17 may for example be a foam, or packed strands or fibres. The core 17 may be formed through an extrusion and/or rolling process into a stable shape. The substrate may be shaped to provide one or more airflow channels. In the particular example of Figure 2, the mouthpiece part 16 defines a plurality of venting holes 22 arranged for example according to the whole perimeter of the mouthpiece part 16 along two axis perpendicular to the substrate axis X. In other words, according to this example, the venting holes 22 are arranged on each wall of the substrate among the substrate lateral walls 13A, 13B and the substrate contact walls 14A, 14B. According to another example, the venting holes 22 are arranged only on the substrate contact walls 14A, 14B or preferably, only on one of the substrate contact walls 14A, 14B. In both examples, the venting holes 22 may be aligned perpendicularly to the substrate axis on the or each corresponding wall of the substrate 12, and can be spaced by a same distance. The venting holes 22 allow fresh air entering inside the substrate 12 to achieve particular vaping/tasting effects.

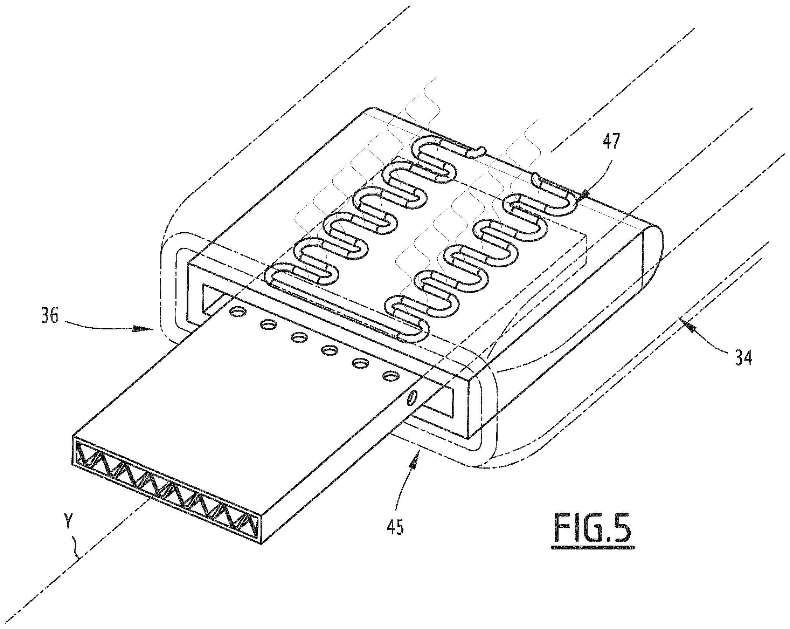

Referring again to Figure 1, the aerosol generating device 10 comprises a device body 30 extending along a device axis Y and forming at least one side wall 40 of the device 10. The device body 30 comprises a mouthpiece 32 and a housing 34 arranged successively according to the device axis Y. According to the first embodiment, the mouthpiece 32 and the housing 34 form two different pieces. Particularly, according to this embodiment, the mouthpiece 32 is designed to be fixed on or be received in an insertion opening 36 formed at one of the ends of the housing 34. This opening 36 extends perpendicularly to the device axis Y as it is shown on Figure 5 where the mouthpiece 32 is removed from the housing 34.

In each cross section, the housing 34 may for example form a substantially rectangular shape with rounded edges. In this case, the housing 34 with the mouthpiece 32 form at least four side walls 40. According to other embodiments, the housing 34 can have a round cross-sectional shape. In this case, it can form with the mouthpiece 32 only one side wall 40. The housing 34 can be sealed at the end opposite to the insertion opening 36 receiving the mouthpiece 32. The housing 34 can be formed from a single piece or several assembled pieces made of any suitable material like aluminium or plastic. In some embodiments, the material of the housing 34 can be a thermally conductive material. In some other embodiments, it can be a thermally insulating material. In some embodiments, the housing 34 can form on the corresponding part of the device side wall 40 one or several openings suitable for arranging control and/or visual elements. For example, such element may comprise control buttons, touch panels, screens, LEDs, etc. Particularly, in the example of Figure 1, the housing 34 forms a slot opening 42 receiving for example a LED indicating at least an ON state of the device 10. It can also indicate for example a battery law state, an error state, etc.

The housing 34 delimits an internal space of the device 10 receiving various elements designed to carry out different functionalities of the device 10. This internal space can for example receive a battery for powering the device 10, a control module for controlling the operation of the device 10, a heating chamber 45 for heating the aerosol generating substrate 12 and at least one heating element 47 for heating the heating chamber 45. Among these elements, only the heating chamber 45 and the heating element 47 will be explained in further detail in reference to Figures 5 to 9.

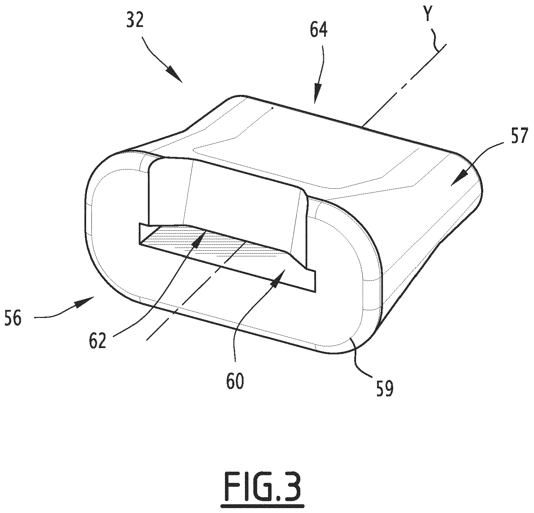

Figure 3 shows in more detail the mouthpiece 32. In reference to this Figure 3, the mouthpiece 32 is delimited by an internal surface 56 intended to face the insertion opening 36 while assembling the mouthpiece 32 with the housing 34, and an external surface 57 intended to form with the housing 34 at least one side wall 40 of the device 10. An external border 59 of the internal surface 56 is designed to be in a tight contact with a part of an internal border of the insertion opening 36 to fix the mouthpiece 32 inside the insertion opening 36. The external surface 57 has an appropriate shape to be in contact with the user's mouth and/or lips. Each side of the external surface 57 can be formed as an extension of the corresponding side of the housing to form an almost continuous side wall 40 of the device 10. Particularly, in this case, a discontinuity can be formed in the transition zone between the mouthpiece 32 and the housing 34.

The mouthpiece 32 is crossed by a through-hole 60 extending along the device axis Y between a recess portion 62 and a flow outlet 64. Particularly, the through-hole 60 is designed to receive the mouthpiece part 16 of the aerosol generating substrate 12 so as the substrate axis X coincides with the device axis Y. Thus, the through-hole 60 has the same cross-sectional shape as the aerosol generating substrate 12 and defines internal dimensions slightly greater than the external dimensions of the mouthpiece part 16 of the aerosol generating substrate 12. Particularly, in the example of the figures, the through-hole 60 defines a rectangular cross-section to be able to receive the mouthpiece part 16 of the aerosol generating substrate 12 shown on Figure 2. In some embodiments, the through-hole 60 may have variable cross-sectional dimensions. For example, the through-hole 60 can have gradually decreasing cross-sectional dimensions (notably the width) from the recess portion 62 to the flow outlet 64. Additionally, the through-hole 60 and the mouthpiece part 16 of the aerosol generating substrate 12 can have the same length measured respectfully according to the device axis Y and the substrate axis X. According to another embodiment, the length of the mouthpiece part 16 of the aerosol generating substrate 12 can be less than the length of the through-hole 60 so as the mouth end 20 of the aerosol generating substrate 12 can be flushed at the flow outlet 64.

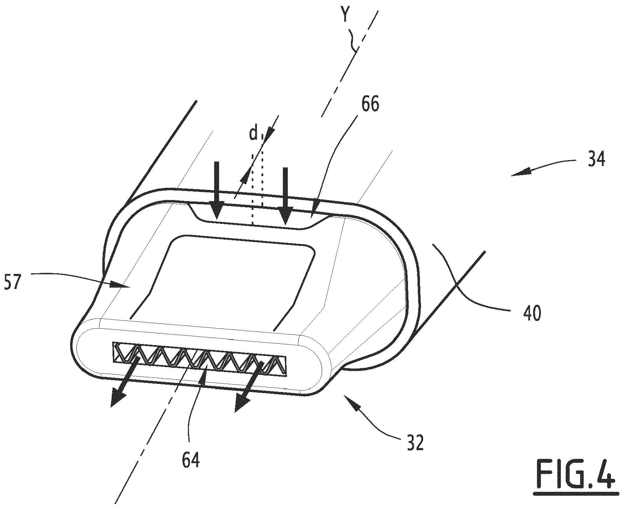

The recess portion 62 corresponds to a cavity formed in both internal and external surfaces 56, 57 of the mouthpiece 32. This cavity can be formed by a first opening extending on the internal surface 56 on one side of the through-hole 60 from the border 59 to this through-hole 60 and a second opening extending on the external surface 57 from the border following d% of the length of the mouthpiece 32 measured according to the device axis Y. The value d can be less than 25, advantageously less than 10 and more advantageously less than 5. Thus, when the mouthpiece 32 is inserted in the insertion opening 36, the recess portion 62 forms an opening 66 forming a flow inlet 66 as shown on Figure 4. In other words, the flow inlet 66 is formed on a side wall 40 of the device 10 in a transition zone between the mouthpiece 32 and the housing 34.

In the embodiment where the aerosol generating substrate 12 comprises the venting holes 22, at least some of these venting holes 22 are arranged to face the flow inlet 66.

In reference to Figures 5 to 9, the heating chamber 45 forms a cup-shaped heating chamber extending along the device axis Y between an open end 70 and a sealed end 71. The heating chamber 45 is designed to receive the heater part 15 of the aerosol generating substrate 12. For this purpose, the heating chamber 45 defines substantially the same cross-sectional shape as the aerosol generating substrate 12. Particularly, in the example of the figures, the heating chamber 45 defines a rectangular cross-sectional shape with two parallel chamber lateral walls 73A, 73B and two parallel chamber contact walls 74A, 74B. The heating chamber 45 also defines a distal wall 75 arranged perpendicularly to the device axis Y and sealing the sealed end 71. Particularly, the distal wall 75 is adjacent to each of the walls 73A, 73B, 74A, 74B so as to seal the chamber at the sealed end 71 and thus, form a cup shape of the chamber opening at the open end 70. Particularly, in this case, the only air permeable opening of the chamber 45 is formed at the open end 70. Each of the walls 73A, 73B, 74A, 74B, 75 can be made from a thermally conductive material like a metal. Additionally, at least some of the walls 73A, 73B, 74A, 74B, 75 or all of these walls can form a single piece.

The internal dimensions of the heating chamber 45 are defined by the length L3 measured according the device axis Y, the width W3 measured as the distance between the chamber lateral walls 73A, 73B and the depth D3 measured as the distance between the chamber contact walls 74A, 74B. These internal dimensions L3, W3, D3 are chosen basing on the external dimensions L2, W, D of the heater part 15 of the aerosol generating substrate 12.

Particularly, the depth D3 of the heating chamber 45 is chosen slightly greater than the depth D of the aerosol generating substrate 12 or substantially equal to this depth D. In this case, the substrate contact walls 14A, 14B can be in contact with the chamber contact walls 74A, 74B when the heater part 15 of the of the aerosol generating substrate 12 is received inside the heating chamber 45. Advantageously, in this case, the chamber contact walls 74A, 74B are in a tight contact with the substrate contact walls 14A, 14B. In some embodiments, the depth D3 of the heating chamber 45 can be even slightly less than the normal depth D of the aerosol generating substrate 12. In this case, the heating chamber 45 and/or the mouthpiece 32 is(are) configured to compress the heater part 15 of the aerosol generating substrate 12 by exerting force on the substrate contact walls 14A, 14B. This makes it possible to improve the tight contact between the corresponding contact walls of the heating chamber 45 and the substrate 12 and thus, to improve heat transfer between these walls.

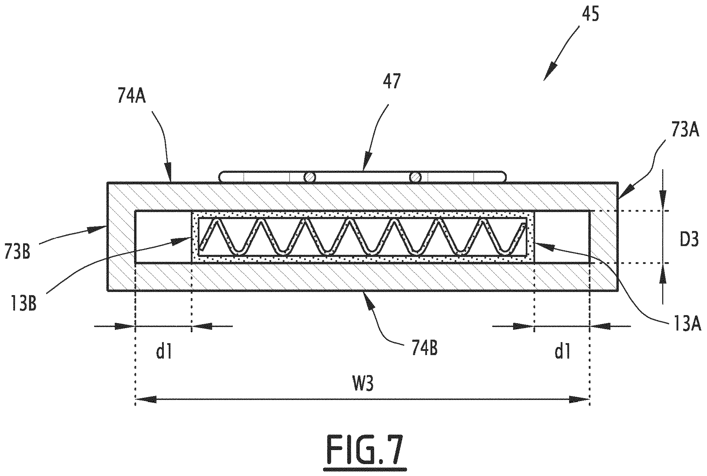

The width W3 of the heating chamber 45 is chosen so as at least one pair of facing lateral walls 73A, 13A or 73B, 13B of the heating chamber 45 and the aerosol generating substrate 12 forms an airflow channel between them. Advantageously, the width W3 of the heating chamber 45 is chosen so as each pair of facing lateral walls 73A, 13A or 73B, 13B of the heating chamber 45 and the aerosol generating substrate 12 forms an airflow channel between them. In other words, the width W3 of the heating chamber 45 is chosen so as to form a distance d1 between each pair of facing lateral walls 73A, 13A or 73B, 13B as it is shown on Figure 7. Particularly, in the example of this Figure, W3=W+2d1. The distance d1 may for example be chosen substantially equal to the depth D3 of the heating chamber 45. In this case, when the heater part 15 of the aerosol generating substrate 12 is inserted in the heating chamber 45, an airflow channel of substantially square cross-section is formed along the device axis Y on either lateral side of the aerosol generating substrate 12. Of course, the distance d1 may be chosen equal to any other value depending on the flow rate required inside the aerosol generating substrate 12 while its heating.

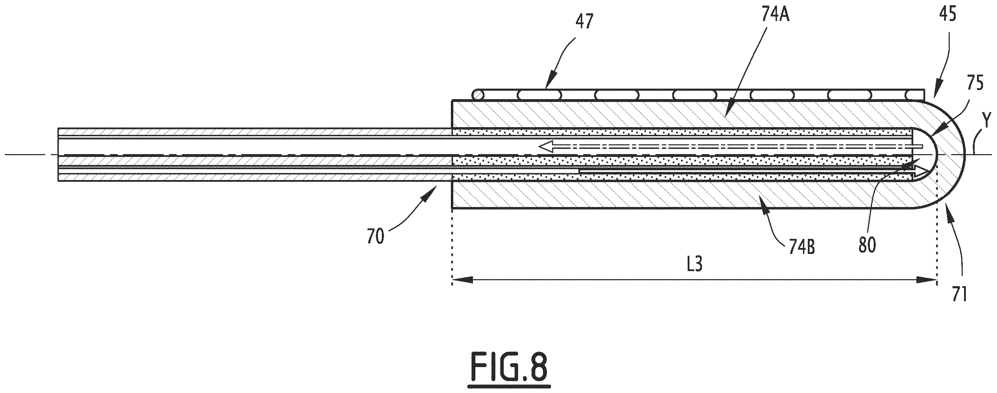

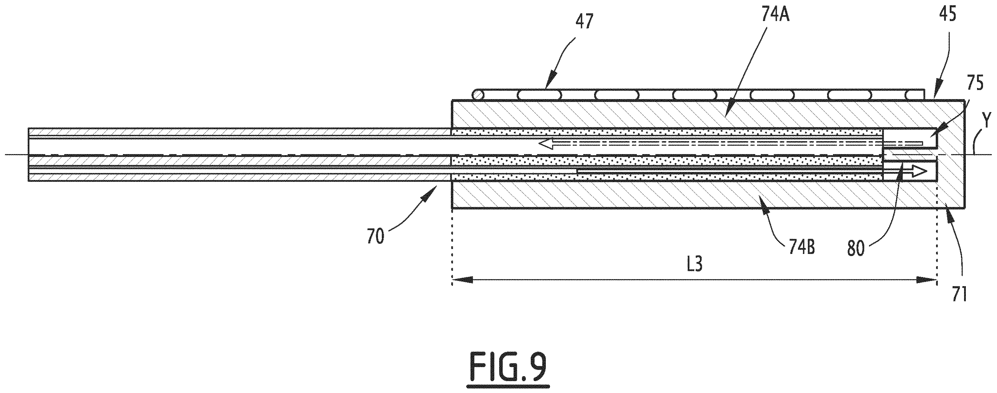

As it is shown on Figures 8 and 9, the length L3 of the heating chamber 45 is chosen so as to receive entirely the heating part 15 of the aerosol generating substrate 12 and form stopping means 80 at the sealed end 71 of the heating chamber 45. In other words, the length L3 is chosen to be strictly greater than the length L2 of the heater part 15 of the aerosol generating substrate 12. The stopping means 80 are configured to prevent contact of the abutting end 18 of the aerosol generating substrate 12 with the distal wall 75 of the heating chamber 45. In other words, the stopping means 80 are configured to create an airflow channel connecting each of the airflow channels formed between the chamber lateral walls 73A, 73B and the aerosol generating substrate 12, with each airflow channel formed inside the aerosol generating substrate 12. In the example of Figure 8, the stopping means 80 are formed by a rounded shape of the distal wall 75. In the example of Figure 9, the stopping means 80 are formed by at least on rib protruding axially from the distal wall 75.

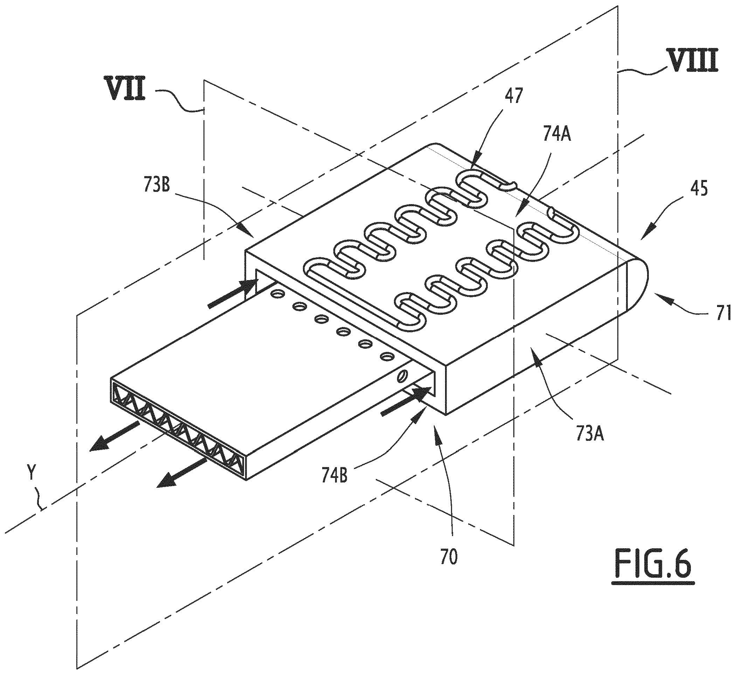

As shown on Figures 5 and 6, the heating element 47 is arranged in contact with one of the chamber contact walls 74A, 74B outside of the heating chamber 45. Particularly, in the example of these Figures, the heating element 47 is arranged adjacent to an outer surface of the chamber contact wall 74A. The heating element 47 may comprise a polyimide film heater extending along substantially the total area of said outer surface of the chamber contact wall 74A or only along a part of this surface. In this last case, said part may form a width substantially equal to the width W of the aerosol generating substrate 12. The heating element 45 is powered by the battery and controlled by the control module of the aerosol generating device 10. In some embodiments, the aerosol generating device 10 may comprise two heating elements 47, each heating element 47 being attached on the outer surface of one of the chamber contact walls 74A, 74B. Additionally, according to some embodiments, the aerosol generating device 10 further comprises an insulator arranged between the or each heating element 47 and an inner surface of the housing 34. The same insulator may also be arranged between an outer surface of each of the chamber lateral walls 73A, 73B and the inner surface of the housing 34.

The operation of the aerosol generating device 10 will now be described. Initially, it is considered that the aerosol generating substrate 12 is extracted from the device 10. In order to insert it, the user first takes off the mouthpiece 32 from the housing 34. Then, the user inserts the heater part 15 of the aerosol generating substrate 12 into the heating chamber 45 until the abutting end 18 of the substrate 12 abuts against the stopping means 80 of the heating chamber 45. Then, the user fixes the mouthpiece 32 on the housing 34 by sliding the mouthpiece part 16 of the aerosol generating substrate 12 inside the through-hole 60 of the mouthpiece 32 and by inserting the mouthpiece 32 in the insertion opening 36 of the housing 34.

Then, the user can activate the operation of the aerosol generating device 10 by actuating for example an ON button or by performing a puff. This creates an airflow in an airflow path formed inside the device between the flow inlet 66 and the flow outlet 64 as it is shown on Figure 4.

The airflow path inside the aerosol generating device 10 comprises an upstream portion extending from the flow inlet 66 until the abutting end 18 of the aerosol generating substrate 12, a transition portion at the sealed end 71 of the heating chamber 45 and a downstream portion extending from said abutting end 18 until the flow outlet 64 through the aerosol generating substrate 12. Particularly, the upstream portion extends through the open end 70 of the heating chamber 45 and then, between each pair of lateral walls 73A, 13A and 73B, 13B of the heating chamber 45 and the aerosol generating substrate 12, as it is shown on Figure 6. Then, the transition portion forms a "U" turn inside the heating chamber 45 as it is shown on Figures 8 and 9 and forces the flow to flow inside the aerosol generating substrate 12. Finally, the downstream portion extends advantageously only inside the aerosol generating substrate 12. In the case when the aerosol generating substrate comprises the venting holes 22, the flow flowing in the downstream portion can further comprise fresh air entered through these holes 22.

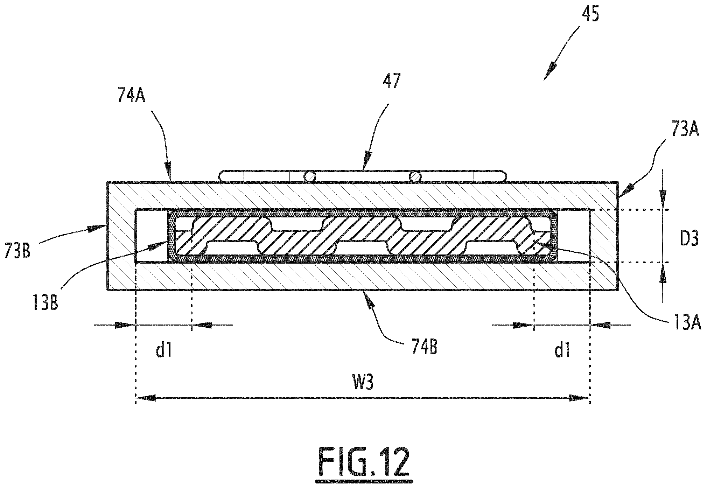

As visible in Figure 12, the heater part 15 of the aerosol generating substrate 12 may have for example a flat-plate shape with slotted grooves on both opposite surfaces. The slotted grooves may form at least part of the downstream portion.

### SECOND EMBODIMENT

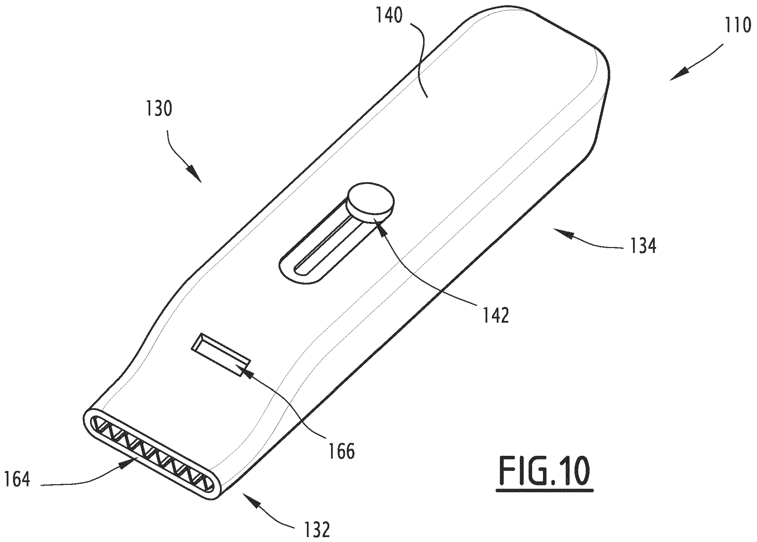

Figure 10 shows an aerosol generating device 110 according to a second embodiment. This aerosol generating device 110 is similar to the aerosol generating device 10 and notably, is configured to operate with the same aerosol generating substrate 12 shown on Figure 2.

An assembly comprises the aerosol generating device 110 and the aerosol generating substrate 12. The aerosol generating substrate 12 is in particular inserted into the aerosol generating device 110. For example, the assembly comprises the aerosol generating substrate 12 being inserted into a heating chamber of the aerosol generating device 110.

The aerosol generating device 110 according to the second embodiment also comprises a device body 130 defining at least one side wall 140 of the device 110 and comprising a mouthpiece 132 and a housing 134 having similar external shapes as respectfully the mouthpiece 32 and the housing 34 explained before in relation with the first embodiment. However, according to the second embodiment, the mouthpiece 132 and the housing 134 form a single piece. In this case, the aerosol generating substrate can for example be loaded directly from an opening 164 of the mouthpiece 132. This opening 164 also forms a flow outlet 164 similar to the flow outlet 64 explained before. The aerosol generating substrate 12 can be extracted from the same opening 164 using for example an internal extracting mechanism which can be actuated by an actuator 142 arranged on the side wall 140.

The or each side wall 140 has for example a smooth external surface and defines a transition zone between the mouthpiece 132 and the housing 134. Furthermore, as in the previous case, the transition zone defines an opening 166 forming a flow inlet 166.

The interior part of the aerosol generating device 110 is similar to the interior part of the aerosol generating device 10 explained above. Particularly, the aerosol generating device 110 comprises the same heating chamber as the heating chamber 45 explained before. As in the previous case, the flow inlet 166 is in a fluid communication with each of the airflow channels formed between the lateral walls of the heating chamber and the aerosol generating substrate. Additionally, as in the previous embodiment, the flow is forced inside the chamber to follow a "U" turn to flow inside the aerosol generating substrate until the flow outlet 164.

The operation of the aerosol generating device 110 is also similar to the operation of the aerosol generating device 10 explained above. The unique difference consists in the way of loading/extracting of the aerosol generating substrate 12.

### THIRD EMBODIMENT

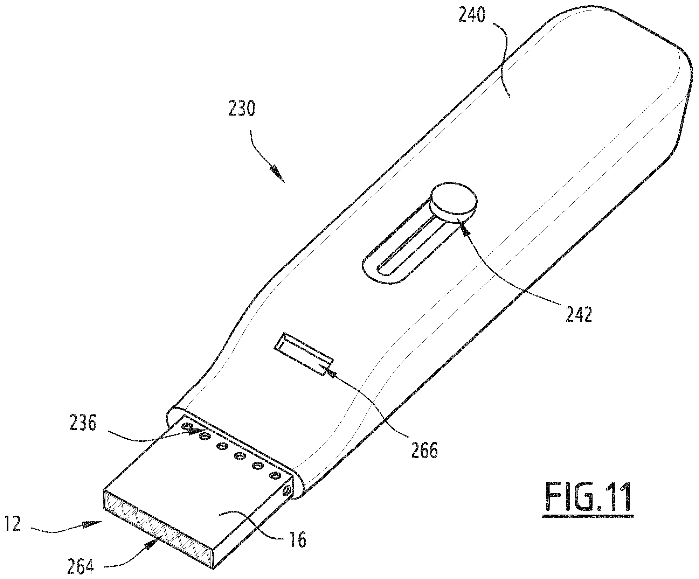

Figure 11 shows an aerosol generating device 210 according to a third embodiment. This aerosol generating device 210 is similar to the aerosol generating device 110 according to the second embodiment and notably, is configured to operate with the same aerosol generating substrate 12 shown on Figure 2.

An assembly comprises the aerosol generating device 210 and the aerosol generating substrate 12. The aerosol generating substrate 12 is in particular inserted into the aerosol generating device 210. For example, the assembly comprises the aerosol generating substrate 12 being inserted into a heating chamber of the aerosol generating device 210.

The aerosol generating device 210 according to the third embodiment also comprises a device body 230 defining at least one side wall 240 of the device 210. Contrary to the previous embodiments, the device body 230 according to the third embodiment does not define a mouthpiece. As it is shown on Figure 11, a mouthpiece is formed by at least a part of the mouthpiece part 16 of the aerosol generating substrate 12.

Particularly, according to this embodiment, the device body 230 forms an insertion opening 236 extending perpendicularly to the device axis Y at one of the ends of the device body 230. As in the second embodiment, the opening 236 is suitable to receive at least the heater part 15 of the aerosol generating substrate 12. Particularly, as in the previous cases, the opening 236 communicates with a heating chamber arranged inside the device body 230. This heating chamber is similar to the heating chamber explained above and notably, is adapted to receive the heater part 15 of the aerosol generating substrate 12.

Contrary to the previous cases, at least a part of the mouthpiece part 16 of the aerosol generating substrate 12 protrudes from the opening 236 forming thus a mouthpiece. In other words, this protruding part of the aerosol generating substrate 12 is designed to be in contact with the user's lips and mouth while using the device 210 and forms a flow outlet 264.

As in the previous cases, a flow inlet 266 is formed on the side wall 240 of the device body 230. This flow inlet 266 is in a fluid communication with each of the airflow channels formed between the lateral walls of the heating chamber and the aerosol generating substrate 12. Additionally, as in the previous cases, the flow is forced inside the chamber to follow a "U" turn to flow inside the aerosol generating substrate 12 until the flow outlet 264.

When the aerosol generating substrate 12 comprises venting holes 22, these venting holes 22 may be arranged on the protruding part of the mouthpiece part 16 of the substrate 12 as it is shown on Figure 11. According to another example, the venting holes 22 are arranged on the part of the mouthpiece part 16 of the substrate 12 which is received inside the device body 230, advantageously to face the flow inlet 266 as in the second embodiment.

As in the second embodiment, an actuator 242 may be arranged on the side wall 240 of the device 210 to facilitate the extraction of the aerosol generating substrate 12. According to another example, no actuator is provided on the side wall and the substrate 12 is extracted by pulling its protruding part.

### FOURTH EMBODIMENT

An aerosol generating device according to a fourth embodimen has the same structure as the aerosol generating device 110 according to the second embodiment, the only difference being in the nature of the substrate 12 that the aerosol generating device according to the fourth embodiment is able to receive.

An assembly comprises the aerosol generating device according to the fourth embodiment and the aerosol generating substrate.

Particularly, the aerosol generating device according to the fourth embodiment can be adapted to receive aerosol generating substrates of different lengths. For example, the aerosol generating device according to the fourth embodiment may be adapted to receive an aerosol generating substrate of a "normal" length, i.e. a substrate similar to the substrate of Figure 2. In this case, this aerosol generating device may operate like the aerosol generating device 110 according to the second embodiment, i.e. it may form a mouthpiece making a part of the device body. The aerosol generating device according to the fourth embodiment may also be adapted to receive an elongated aerosol generating substrate. In this case, the substrate can protrude from the device body and form a mouthpiece. In other words, in this case, the aerosol generating device according to the fourth embodiment may operate as the aerosol generating device 210 according to the third embodiment.

## Claims

1. An aerosol generating device (10; 110; 210) configured to operate with an aerosol generating substrate (12) and comprising:
- a device body (30; 130; 230) extending along a device axis (Y) and defining at least one side wall (40; 140; 240) extending along the device axis (Y);
- a cup-shaped heating chamber (45) defining an open end (70) and a sealed end (71) opposite to the open end (70), the sealed end (71) being air impermeable, the heating chamber (45) being configured to receive a heater part (15) of the aerosol generating substrate (12) through the open end (70);
- an airflow path extending between a flow inlet (66; 166; 266) and an flow outlet (64; 164; 264) through the open end (70) of the heating chamber (45),
wherein the airflow path comprises an upstream portion extending from the flow inlet (66; 166; 266) until an abutting end (18) of the aerosol generating substrate (12), and a downstream portion extending from said abutting (18) end until the flow outlet (64; 164; 264) through the aerosol generating substrate (12), when the aerosol generating substrate (12) is inserted into the heating chamber (45),
**characterized in that** the flow inlet (66; 166) is arranged on the side wall (40; 140; 240) of the device body (30; 130; 230).

2. The aerosol generating device (10; 110) according to claim 1, wherein the device body (30; 130) comprises a mouthpiece (32; 132) and a housing (34; 134) extending successively along the device body (30; 130), the mouthpiece (32; 132) defining a through-hole (60) configured to receive a mouthpiece part (16) of the aerosol generating substrate (12).

3. The aerosol generating device (10; 110) according to claim 2, wherein the flow inlet (66; 166) is formed in a transition zone between the mouthpiece (32; 132) and the housing (34; 134).

4. The aerosol generating device (110) according to claim 3, wherein the mouthpiece (132) and the housing (134) form a single piece, the transition zone being formed by a smooth surface extending from the mouthpiece (132) to the housing (134).

5. The aerosol generating device (10) according to claim 3, wherein the housing (34) defines an insertion opening (36) extending perpendicularly to the device axis (Y) and configured to receive the mouthpiece (32).

6. The aerosol generating device (10) according to claim 5, wherein the mouthpiece (32) defines a recess portion (62) forming the flow inlet (66) when the mouthpiece (32) is inserted into the insertion opening (36) of the housing (34).

7. The aerosol generating device (10) according to claim 6, wherein the through-hole (60) of the mouthpiece opens to the recess portion (62).

8. The aerosol generating device (210) according to claim 1, wherein the device body (230) defines an insertion opening (236) on one of its ends suitable to receive a part of the aerosol generating substrate (12);
when received in the insertion opening (236), the aerosol generating substrate (12) forming a protruding part from the device body (230), said protruding part forming a mouthpiece.

9. The aerosol generating device (10; 110; 210) according to any one of the preceding claims, wherein the flow inlet (66; 166; 266) is arranged to face ventilation holes (22) of the aerosol generating substrate (12).

10. The aerosol generating device (10; 110; 210) according to any one of the preceding claims, wherein at least a part of the upstream portion of the airflow path extends through the heating chamber (45) outside the aerosol generating substrate (12),
preferably, the upstream portion of the airflow path extends through the open end (70) of the heating chamber (45).

11. The aerosol generating device (10; 110; 210) according to any one of the preceding claims, wherein the heating chamber (45) extends along the device axis (Y) and defines a rectangular cross-sectional shape having a pair of chamber lateral walls (73A, 73B) facing each other and a pair of chamber contact walls (74A, 74B) facing each other and intended to be in contact with substrate contact walls (14A, 14B) of the aerosol generating substrate (12).

12. The aerosol generating device (10; 110; 210) according to claims 10 and 11, wherein said part of the upstream portion of the airflow path extends between at least one chamber lateral wall (73A, 73B) and the aerosol generating substrate (12).

13. The aerosol generating device (10; 110; 210) according to claim 12, wherein the sealed end (71) of the heating chamber (45) forms a transition portion between the upstream portion and the downstream portion of the airflow path.

14. The aerosol generating device (10; 110; 210) according to claim 13, wherein the sealed end (71) defines stopping means (80) preventing contact of the abutting end (18) of the aerosol generating substrate (12) with a distal wall (75) of the heating chamber (45) sealing it at the sealed end (71);
preferably, the stopping means (80) being formed by a rounded shape of the distal wall (75) or a rib extending from the distal wall (75).

15. Assembly comprising an aerosol generating device (10; 110; 210) according to any one of the preceding claims, and furthermore comprising an aerosol generating substrate (12).

## Patentansprüche

1. Aerosolerzeugungsvorrichtung (10; 110; 210), die so konfiguriert ist, dass sie mit einem Aerosolerzeugungssubstrat (12) arbeitet, und Folgendes umfasst:
- einen Vorrichtungskörper (30; 130; 230), der sich entlang einer Vorrichtungsachse (Y) erstreckt und mindestens eine Seitenwand (40; 140; 240) definiert, die sich entlang der Vorrichtungsachse (Y) erstreckt;
- eine becherförmige Heizkammer (45), die ein offenes Ende (70) und ein abgedichtetes Ende (71) gegenüber dem offenen Ende (70) definiert, wobei das abgedichtete Ende (71) luftundurchlässig ist, die Heizkammer (45) so konfiguriert ist, dass sie einen Heizerteil (15) des Aerosolerzeugungssubstrats (12) durch das offene Ende (70) aufnimmt;
- einen Luftstromweg, der sich zwischen einem Strömungseinlass (66; 166; 266) und einem Strömungsauslass (64; 164; 264) durch das offene Ende (70) der Heizkammer (45) erstreckt,
wobei der Luftströmungsweg einen stromaufwärts gelegenen Abschnitt umfasst, der sich von dem Strömungseinlass (66; 166; 266) bis zu einem Anschlagende (18) des Aerosolerzeugungssubstrats (12) erstreckt, und einen stromabwärts gelegenen Abschnitt, der sich von dem Anschlagende (18) bis zu dem Strömungsauslass (64; 164; 264) durch das Aerosolerzeugungssubstrat (12) erstreckt, wenn das Aerosolerzeugungssubstrat (12) in die Heizkammer (45) eingesetzt ist,
**dadurch gekennzeichnet, dass** der Strömungseinlass (66; 166) an der Seitenwand (40; 140; 240) des Vorrichtungskörpers (30; 130; 230) angeordnet ist.

2. Aerosolerzeugungsvorrichtung (10; 110) nach Anspruch 1, wobei der Vorrichtungskörper (30; 130) ein Mundstück (32; 132) und ein Gehäuse (34; 134) umfasst, die sich nacheinander entlang des Vorrichtungskörpers (30; 130) erstrecken, wobei das Mundstück (32; 132) ein Durchgangsloch (60) definiert, das so konfiguriert ist, dass es ein Mundstückteil (16) des Aerosolerzeugungssubstrats (12) aufnimmt.

3. Aerosolerzeugungsvorrichtung (10; 110) nach Anspruch 2, wobei der Strömungseinlass (66; 166) in einem Übergangsbereich zwischen dem Mundstück (32; 132) und dem Gehäuse (34; 134) ausgebildet ist.

4. Aerosolerzeugungsvorrichtung (110) nach Anspruch 3, bei der das Mundstück (132) und das Gehäuse (134) ein einziges Stück bilden, wobei der Übergangsbereich durch eine glatte Oberfläche gebildet wird, die sich vom Mundstück (132) zum Gehäuse (134) erstreckt.

5. Aerosolerzeugungsvorrichtung (10) nach Anspruch 3, wobei das Gehäuse (34) eine Einführungsöffnung (36) definiert, die sich senkrecht zur Vorrichtungsachse (Y) erstreckt und zur Aufnahme des Mundstücks (32) konfiguriert ist.

6. Aerosolerzeugungsvorrichtung (10) nach Anspruch 5, wobei das Mundstück (32) einen Aussparungsabschnitt (62) definiert, der den Strömungseinlass (66) bildet, wenn das Mundstück (32) in die Einführungsöffnung (36) des Gehäuses (34) eingeführt wird.

7. Aerosolerzeugungsvorrichtung (10) nach Anspruch 6, wobei sich das Durchgangsloch (60) des Mundstücks in den Aussparungsabschnitt (62) öffnet.

8. Aerosolerzeugungsvorrichtung (210) nach Anspruch 1, wobei der Vorrichtungskörper (230) an einem seiner Enden eine Einführungsöffnung (236) definiert, die geeignet ist, einen Teil des Aerosolerzeugungssubstrats (12) aufzunehmen;
wobei, wenn es in der Einführungsöffnung (236) aufgenommen ist, das Aerosolerzeugungsssubstrat (12) einen aus dem Vorrichtungskörper (230) hervorstehenden Teil bildet, wobei der hervorstehende Teil ein Mundstück bildet.

9. Aerosolerzeugungsvorrichtung (10; 110; 210) nach einem der vorhergehenden Ansprüche, wobei der Strömungseinlass (66; 166; 266) so angeordnet ist, dass er den Belüftungslöchern (22) des Aerosolerzeugungssubstrats (12) gegenüberliegt.

10. Aerosolerzeugungsvorrichtung (10; 110; 210) nach einem der vorhergehenden Ansprüche, wobei sich mindestens ein Teil des stromaufwärts gelegenen Abschnitts des Luftströmungswegs durch die Heizkammer (45) außerhalb des Aerosolerzeugungssubstrats (12) erstreckt,
wobei sich der stromaufwärts gelegene Teil des Luftströmungswegs vorzugsweise durch das offene Ende (70) der Heizkammer (45) erstreckt.

11. Aerosolerzeugungsvorrichtung (10; 110; 210) nach einem der vorangehenden Ansprüche, wobei sich die Heizkammer (45) entlang der Vorrichtungsachse (Y) erstreckt und eine rechteckige Querschnittsform mit einem Paar einander zugewandter Kammerseitenwände (73A, 73B) und einem Paar einander zugewandter Kammerkontaktwände (74A, 74B) definiert, die dazu bestimmt sind, mit Substratkontaktwänden (14A, 14B) des Aerosolerzeugungssubstrats (12) in Kontakt zu stehen.

12. Aerosolerzeugungsvorrichtung (10; 110; 210) nach einem der Ansprüche 10 und 11, wobei sich der Teil des stromaufwärts gelegenen Abschnitts des Luftströmungswegs zwischen mindestens einer Kammerseitenwand (73A, 73B) und dem Aerosolerzeugungssubstrat (12) erstreckt.

13. Aerosolerzeugungsvorrichtung (10; 110; 210) nach Anspruch 12, wobei das abgedichtete Ende (71) der Heizkammer (45) einen Übergangsabschnitt zwischen dem stromaufwärts gelegenen Abschnitt und dem stromabwärts gelegenen Abschnitt des Luftströmungswegs bildet.

14. Aerosolerzeugungsvorrichtung (10; 110; 210) nach Anspruch 13, wobei das abgedichtete Ende (71) eine Anschlageinrichtung (80) definiert, die den Kontakt des Anschlagendes (18) des Aerosolerzeugungssubstrats (12) mit einer distalen Wand (75) der Heizkammer (45) verhindert, die es an dem abgedichteten Ende (71) abdichtet;
Wobei das Anschlagmittel (80) vorzugsweise durch eine abgerundete Form der distalen Wand (75) oder eine von der distalen Wand (75) ausgehende Rippe gebildet wird.

15. Baugruppe, umfassend eine Aerosolerzeugungsvorrichtung (10; 110; 210) nach einem der vorhergehenden Ansprüche und ferner umfassend ein Aerosolerzeugungssubstrat (12).

## Revendications

1. Dispositif de génération d'aérosol (10 ; 110 ; 210) configuré pour fonctionner avec un substrat de génération d'aérosol (12) et comprenant :
- un corps de dispositif (30 ; 130 ; 230) s'étendant le long d'un axe du dispositif (Y) et définissant au moins une paroi latérale (40 ; 140 ; 240) s'étendant le long de l'axe du dispositif (Y) ;
- une chambre de chauffe en forme de coupelle (45) définissant une extrémité ouverte (70) et une extrémité scellée (71) opposée à l'extrémité ouverte (70), l'extrémité scellée (71) étant imperméable à l'air, la chambre de chauffage (45) étant configurée pour recevoir une partie chauffante (15) du substrat de génération d'aérosol (12) à travers l'extrémité ouverte (70) ;
- une voie de circulation d'air s'étendant entre une entrée de flux (66 ; 166 ; 266) et une sortie de flux (64 ; 164 ; 264) à travers l'extrémité ouverte (70) de la chambre de chauffe (45),
dans lequel la voie de circulation d'air comprend une partie en amont s'étendant de l'entrée du flux (66 ; 166 ; 266) jusqu'à une extrémité en butée (18) du substrat de génération d'aérosol (12), et une partie en aval s'étendant de ladite extrémité en butée (18) jusqu'à la sortie du flux (64 ; 164 ; 264) à travers le substrat de génération d'aérosol (12), lorsque le substrat de génération d'aérosol (12) est inséré dans la chambre de chauffe (45),
**caractérisé en ce que** l'entrée de flux (66 ; 166) est disposée sur la paroi latérale (40 ; 140 ; 240) du corps du dispositif (30 ; 130 ; 230).

2. Dispositif de génération d'aérosol (10 ; 110) selon la revendication 1, dans lequel le corps du dispositif (30 ; 130) comprend un embout (32 ; 132) et un boîtier (34 ; 134) s'étendant successivement le long du corps du dispositif (30 ; 130), l'embout (32 ; 132) définissant un trou traversant (60) configuré pour recevoir une partie de l'embout (16) du substrat de génération d'aérosol (12).

3. Dispositif de génération d'aérosol (10 ; 110) selon la revendication 2, dans lequel l'entrée de flux (66 ; 166) est formée dans une zone de transition entre l'embout (32 ; 132) et le boîtier (34 ; 134).

4. Dispositif de génération d'aérosol (110) selon la revendication 3, dans lequel l'embout (132) et le boîtier (134) forment une seule pièce, la zone de transition étant formée par une surface lisse s'étendant de l'embout (132) au boîtier (134).

5. Dispositif de génération d'aérosol (10) selon la revendication 3, dans lequel le boîtier (34) définit une ouverture d'insertion (36) s'étendant perpendiculairement à l'axe du dispositif (Y) et configurée pour recevoir l'embout (32).

6. Dispositif de génération d'aérosol (10) selon la revendication 5, dans lequel l'embout (32) définit une partie évidée (62) formant l'entrée du flux (66) lorsque l'embout (32) est inséré dans l'ouverture d'insertion (36) du boîtier (34).

7. Dispositif de génération d'aérosol (10) selon la revendication 6, dans lequel le trou traversant (60) de l'embout débouche sur la partie évidée (62).

8. Dispositif de génération d'aérosol (210) selon la revendication 1, dans lequel le corps du dispositif (230) définit une ouverture d'insertion (236) sur l'une de ses extrémités, apte à recevoir une partie du substrat de génération d'aérosol (12) ;
lorsqu'il est reçu dans l'ouverture d'insertion (236), le substrat de génération d'aérosol (12) forme une partie saillante par rapport au corps du dispositif (230), ladite partie saillante formant un embout.

9. Dispositif de génération d'aérosol (10 ; 110 ; 210) selon l'une quelconque des revendications précédentes, dans lequel l'entrée du flux (66 ; 166 ; 266) est disposée de manière à faire face aux orifices de ventilation (22) du substrat de génération d'aérosol (12).

10. Dispositif de génération d'aérosol (10 ; 110 ; 210) selon l'une quelconque des revendications précédentes, dans lequel au moins une partie de la partie amont de la voie de circulation d'air s'étend à travers la chambre de chauffe (45) à l'extérieur du substrat de génération d'aérosol (12),
de préférence, la partie amont de la voie de circulation d'air s'étend à travers l'extrémité ouverte (70) de la chambre de chauffe (45).

11. Dispositif de génération d'aérosol (10 ; 110 ; 210) selon l'une quelconque des revendications précédentes, dans lequel la chambre de chauffe (45) s'étend le long de l'axe du dispositif (Y) et définit une forme de section transversale rectangulaire ayant une paire de parois latérales de chambre (73A, 73B) se faisant face et une paire de parois de contact de chambre (74A, 74B) se faisant face et destinées à être en contact avec les parois de contact du substrat (14A, 14B) du substrat de génération d'aérosol (12).

12. Dispositif de génération d'aérosol (10 ; 110 ; 210) selon les revendications 10 et 11, dans lequel ladite partie de la partie amont de la voie de circulation d'air s'étend entre au moins une paroi latérale de la chambre (73A, 73B) et le substrat de génération d'aérosol (12).

13. Dispositif de génération d'aérosol (10 ; 110 ; 210) selon la revendication 12, dans lequel l'extrémité scellée (71) de la chambre de chauffe (45) forme une partie de transition entre la partie amont et la partie aval de la voie de circulation d'air.

14. Dispositif de génération d'aérosol (10 ; 110 ; 210) selon la revendication 13, dans lequel l'extrémité scellée (71) définit des moyens d'arrêt (80) empêchant le contact de l'extrémité de butée (18) du substrat de génération d'aérosol (12) avec une paroi distale (75) de la chambre de chauffe (45) qui la scelle au niveau de l'extrémité scellée (71) ;
de préférence, les moyens d'arrêt (80) étant formés par une forme arrondie de la paroi distale (75) ou par une nervure s'étendant à partir de la paroi distale (75).

15. Ensemble comprenant un dispositif de génération d'aérosol (10 ; 110 ; 210) selon l'une quelconque des revendications précédentes, et comprenant en outre un substrat de génération d'aérosol (12).
